# EUROPEAN PATENT SPECIFICATION

(11) **EP 2 361 551 B1**
(45) Date of publication and mention of the grant of the patent: **02.09.2015**
(21) Application number: 11153002.8
(22) Date of filing: 02.02.2011
(51) Int. Cl.: A61B 5/053, G01G 19/44, G01G 21/28, G01G 23/18

(54) **Living body index measurement apparatus**
Vorrichtung zur Messung des Index eines lebenden Körpers
Appareil de mesure d'un indice de corps vivant

(30) Priority: 26.02.2010 JP 2010043409
(43) Date of publication of application: 31.08.2011
(73) Proprietor: Tanita Corporation, Tokyo 174-8630 (JP)
(72) Inventor: Kosaka, Hiroki, Itabashi-ku Tokyo 174-8630 (JP); Hatakeyama, Minoru, Itabashi-ku Tokyo 174-8630 (JP); Saito, Atsushi, Itabashi-ku Tokyo 174-8630 (JP)
(74) Representative: Haley, Stephen

(56) References cited:
- EP-A2- 2 323 057
- CN-Y- 2 886 521
- DE-U1- 20 316 626
- JP-A- 9 238 926
- JP-A- 62 028 619
- JP-A- 2009 002 924
- US-A1- 2005 187 486
- US-B1- 6 369 337
- US-B2- 7 550 682

## Description

### BACKGROUND OF THE INVENTION

### 1. Field of the Invention

The present invention relates to a living body index measurement apparatus such as a body composition measuring apparatus.

### 2. Description of Related Art

A body composition measuring apparatus (body composition monitor), such as is disclosed in Japanese Patent Application Laid-Open Publication No. 2010-11906, is provided with a base plate unit disposed on a floor surface. The base plate unit has a platform. An upright pillar extends from the base plate unit in the vertical direction. A display unit is disposed at a predetermined height of the pillar. Displayed on the screen of the display unit are living body information such as weight and body fat percentage. Users stand upright on the platform during measurement. Users can check the weight or body fat percentage displayed on the display unit while standing up.

The pillar is fixed at the base plate unit. The display unit is embedded at the top end portion of the pillar. A PC (Personal Computer) board is built into the display unit. The PC board of the display unit calculates living body indices such as body fat percentage based on weight and bioelectrical impedance. The pillar is not easily detachable from the base plate unit. The display unit and the PC board cannot be easily repaired or replaced on site. It is therefore troublesome to transport the whole body composition measurement apparatus for repairs and replacement.

CN2886521Y and JP 2009 002924A both disclose apparatuses in which the the cable plate unit is detachably mounted to the pillar, which is in turn detachably mounted to the display unit.

### SUMMARY OF THE INVENTION

Accordingly, the present invention has, as an object, to provide a living body index measurement apparatus that can contribute to increased workability for repairs and replacements.
To achieve the above object, the present invention provides a living body index measurement apparatus for measuring a living body index of a human subject and for displaying information on the measured living body index, comprising:
a base plate unit having a platform on which the human subject stands while measurement is taken;
a calculation processing circuit that is built in the base plate unit and for calculating a living body index of the human subject based on a measurement value obtained from the human subject;
a pillar that stands upright from the base plate unit, with a bottom end of the pillar being connected detachably to the base plate unit; and
a display unit that is detachably mounted at a top end of the pillar and for displaying information including the living body index, the display unit having a processor; and
a cable for connecting between the display unit and the base plate unit, with one end of the cable having a third connector for connecting, in a detachable manner, to the first connector of the first interface board of the display unit.
characterised in that:
   the pillar has a back side in which a groove is formed, the groove extending in the vertical direction,
   the display unit is received in a recess at a top end of the pillar in a detachable manner, and includes a first interface board having a first connector connected to the processor,
   a second interface board is built in the base plate unit, the second interface board having a second connector connected to the calculation processing circuit in the base plate unit,
   the cable has an end having a third connector detachably connected to the first connector of the first interface board in the display unit, the cable having another end having a fourth connector detachably connected to the second connector of the second interface board in the base plate unit, the cable extending along the external surface of the pillar and accommodated in the groove of the pillar.

In the living body index measurement apparatus, the display unit can be detached from the pillar. Furthermore, the calculation process circuit is built into the base plate unit. Therefore, the display unit can be separately replaced on site. A component of the display unit can be replaced or repaired at a place other than the installed site. The entire body of a living body index measurement apparatus does not have to be carried for such repairs and replacements. Therefore, the work burden is reduced, and the workability is enhanced in repairs and replacements. It becomes relatively easy to update a component of the display unit, and the entire display unit can be replaced with one having a new function.

The living body index measurement apparatus further has a cable which extends along the external surface of the pillar, the cable connecting between the base plate unit and the display unit. The pillar of the living body index measurement apparatus can be detached from the base plate unit. The connection between the display unit and the base plate unit can be maintained by the action of the cable even if the pillar is detached. Therefore, a user can continue using the display unit. A measurer who is not the user standing on the platform can separate the display of the display unit from the view of the user.

In a preferred embodiment, the living body index measurement apparatus may have a finger screw for connecting the pillar and the base plate unit, and the pillar may be connected to the base plate unit in such a manner that it is attachable to or detachable from the base plate unit by means of the finger screw. According to this embodiment, the pillar can be easily attached and detached by means of a finger screw with no need for any special equipment.

The living body index measurement apparatus may additionally have a weight-to-signal conversion device which is built within the base plate unit and for generating a signal that changes in accordance with a weight of a living body which is on the platform; a weight measurement circuit which is built within the base plate unit and for generating a weight signal indicating a value of the weight based on the signal from the weigh-to-signal conversion device; plural current electrodes for supplying a current to the living body; plural voltage electrodes for contacting the living body; and an impedance measurement circuit which is built within the base plate unit and for generating an impedance signal indicating an impedance of the living body based on a voltage given to the voltage electrode and the current. In this case, the calculation processing circuit may calculate a living body index for the living body based on the weight signal and the impedance signal.

According to this living body index measurement apparatus, the weight measurement circuit and the impedance measurement circuit are built in the base plate unit in addition to the calculation processing circuit. Therefore, the main function of a living body index measurement is independent from the display unit and is provided in the base plate unit. In the display unit, minimum required components are, for example, an interlace for exchanging signals with the base plate unit, and a control board for Display Therefore, if there is a problem with the living body index measurement apparatus, it is possible to easily identify whether the problem exists in the base plate unit or in the display unit. When the location of the problem is identified, the display unit alone or the base plate unit alone can be replaced.

### BRIEF DESCRIPTION OF THE DRAWINGS

Fig. 1 is a perspective view showing an external view of a body composition measurement apparatus according to an embodiment of the present invention.
Fig. 2 is an exploded perspective view of the body composition measurement apparatus.
Fig. 3 is a block diagram schematically showing a control system of the body composition measurement apparatus.
Fig. 4 is a block diagram schematically showing an electric system of the body composition measurement apparatus.
Fig. 5 is a perspective view schematically showing the body composition measurement apparatus for which a display unit is detached from a pillar.
Fig. 6 is a perspective view schematically showing the body composition measurement apparatus for which the pillar is detached from a base plate unit.
Fig. 7 is an enlarged view of the pillar.

### DETAILED DESCRIPTION OF PREFERRED EMBODIMENTS

In the following, description will be given of an embodiment of the present invention with reference to the attached drawings.

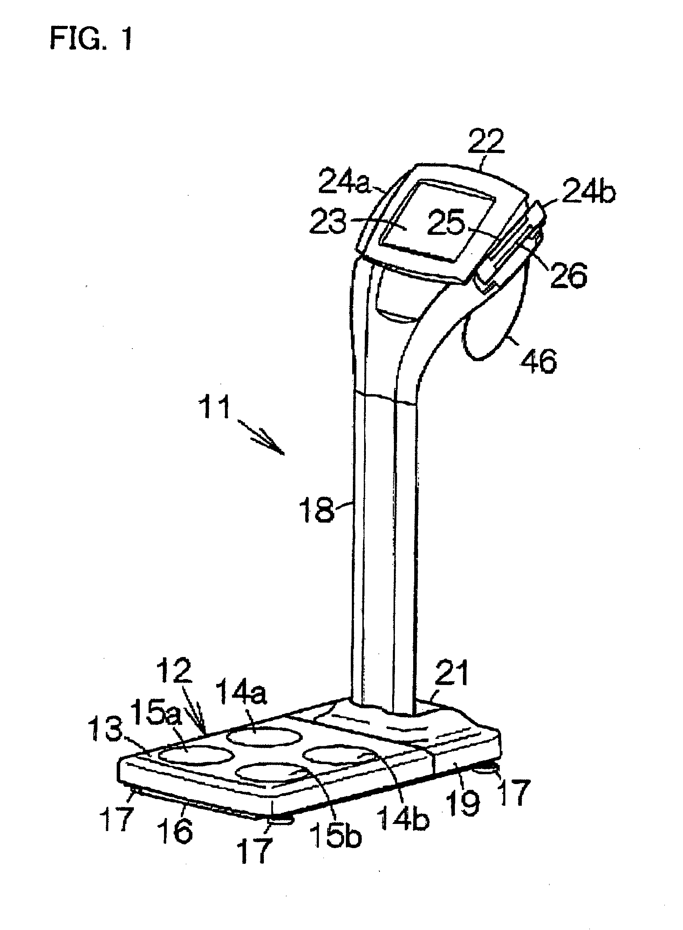

Fig. 1 shows a body composition measurement apparatus (living body index measurement apparatus) 11 according to an embodiment of the present invention. As shown in the figure, the body composition measurement apparatus 11 has a flat base plate unit 12. Base plate unit 12 has a platform; 13. Although not shown, platform 13 has a box part made of a steel plate and a resin cover mounted to the exterior of the box part. The resin cover serves as an outer package of the platform, and also insulates a part made of a steel plate and electrode 14a, 14b, 15a, 15b (described later).

Platform 13 (the resin cover outside platform 13) has, for example, a horizontal flat surface. The flat surface is provided with a right current electrode 14a and a left current electrode 14b, and with a right voltage electrode 15a and a left voltage electrode 15b. Current electrodes 14a and 14b and voltage electrodes 15a and l5b are made of, for example, metallic material such as stainless steel or another type of conductive material. Description will be given below of the details of current electrodes 14a and 14b and voltage electrodes 15a and 15b. A user of body composition measurement apparatus 11 stands upright on platform 13. The sole of the left foot of the user touches left current electrode 14a and left voltage electrode 15a, and the sole of the right leg of the user standing on platform 13 touches right current electrode 14b and right voltage electrode 15b.

Base plate unit 12 has a base 16. Base 16 is made of, for example, a rigid body such as of stainless steel. Base 16 is shaped to be approximately rectangular in plantar view. Four legs 17 are fixed at respective four corners of the rectangle, although there are shown three legs 17 in Fig. 1. Base 16 is supported above the floor surface by legs 17. Legs 17 can be displaced with respect to base 16 to a direction perpendicular to the floor surface. The distance between the undersides of legs 17 and platform 13 can be thus adapted, by way of which the horizontal attitude of platform 13 is established. When the horizontal attitude is established, the flat surface of platform 13 bisects the gravity direction at right angles. The displacement of legs 17 may be implemented, for example, by the action of a threaded shaft having a shaft center in the direction of gravity.

Fixed to base plate unit 12 to the front side of platform 13 is a pillar (supporting, upstanding member) 18. Pillar 18 stands in a vertical direction. Downward of the vertical direction corresponds to the direction of gravity. Provided at the front side of platform 13 and in the front and the back of pillar 18 are front cover 19 and rear cover 21 attached to base plate unit 12.

Display unit 22 is detachably connected to pillar 18 at a predetermined level (height). Display unit 22 is spatially distant from platform 13 in the vertical direction. Touch screen panel 23 is built in display unit 22. Various types of pieces of information are displayed on the screen of touch screen panel 23. Such a piece of information or pieces of information include at least one of living body information such as weight, body fat percentage, muscle mass, total body water (amount of body water), basal metabolic rate (BMR), bone mass, visceral fat level, or metabolic age (a number that is derived from comparing a BMR with the BMR average of a chronological age group of a human subject).

Touch screen panel 23 concurrently serves as an input device. Instructions or data can be input at display unit 22 in accordance with a touch operation of a user at touch screen panel 23. The level, or height, of display unit 22 may be determined depending on the height of a user. The visibility to the user may be sufficiently maintained depending on the level of display unit 22.

Right handheld electrode unit 24a and left handheld electrode unit 24b are provided (supported) at pillar 18 at a predetermined level by being attachable and detachable from pillar 18. Handheld electrode units 24a and 24b are spatially distant from platform 13 in the vertical direction. Current electrode 25 and voltage electrode 26 are mounted on left handheld electrode unit 24a, as described below. Similarly, current electrode 25 and voltage electrode 26 are mounted to right handheld electrode unit 24b. In a case in which a user of body composition measurement apparatus 11 grasps left handheld electrode unit 24a by the left hand, current electrode 25 and voltage electrode 26 come into contact with the palm of the left hand. Similarly, in a case in which a user grasps right handheld electrode unit 24b by the right hand, current electrode 25 and voltage electrode 26 come into contact with the palm of the right hand.

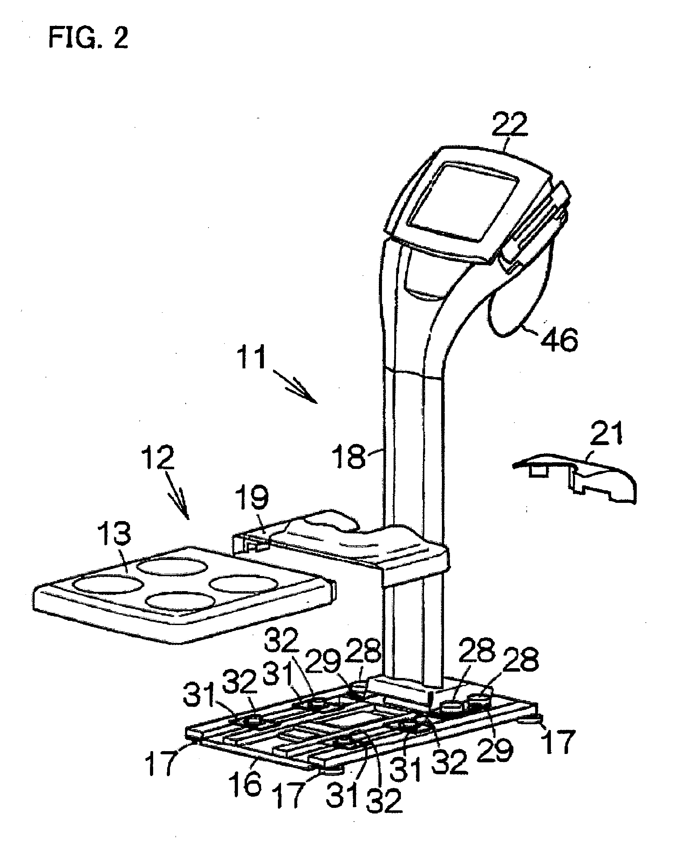

As shown in Fig. 2, pillar 18 is connected to base 16 by being detachable therefrom. Four finger screws 28 are used for this connection. Three finger screws 28 are shown in Fig. 2. Formed at the foot of pillar 18 are right and left flat plates 29 overlaid on the horizontal plane of base 16. The center shaft of finger screw 28 extends in the vertical direction. The shaft of finger screw 28 is screwed into base 16. Flat plate 29 is inserted between the head of finger screw 28 and base 16. Pillar 18 can be easily attached and removed by the action of finger screw 28 with no access to the particular equipment. The number of finger screws 28 is not limited to four but may be any number. For example, one finger screw 28 may be used.

Four load cell units 31 are fixed at base 16. Load cell unit 31 has a load transmission part 32 which is displaced in the vertical direction. The load given to platform 13 is transmitted to four load transmission parts 32 and further to four load cells (not shown). The load given to each load transmission part 32 is converted into an electric signal at respective load cells. The electric signal specifies the weight that acts on load transmission part 32. Thus, load cell unit 31 serves as a weight-to-signal conversion device.

The above-described front cover 19, rear cover 21, and platform 13 are supported by base 16. Front cover 19 and rear cover 21 are made of, for example, synthetic resin. Front cover 19 and rear cover 21 are connected to base 16 by being attachable and detachable by the action of their own elastic force. Thus, front cover 19 and rear cover 21 are placed over the foot of pillar 18 from the front and the back sides of pillar 18. As a result, base plate unit 12 appears to be perfectly shaped and is esthetically appealing. Platform 13 is connected to load transmission parts 32 of load cell unit 31. For this connection, a screw (not shown), for example, is used. Platform 13 is supported by a load cell which is built within base plate unit 12. In a case in which a user stands upright on platform 13, each load transmission part 32 moves vertically in accordance with the weight of the user. Each load cell thus outputs a predetermined electric signal depending on the user's weight.

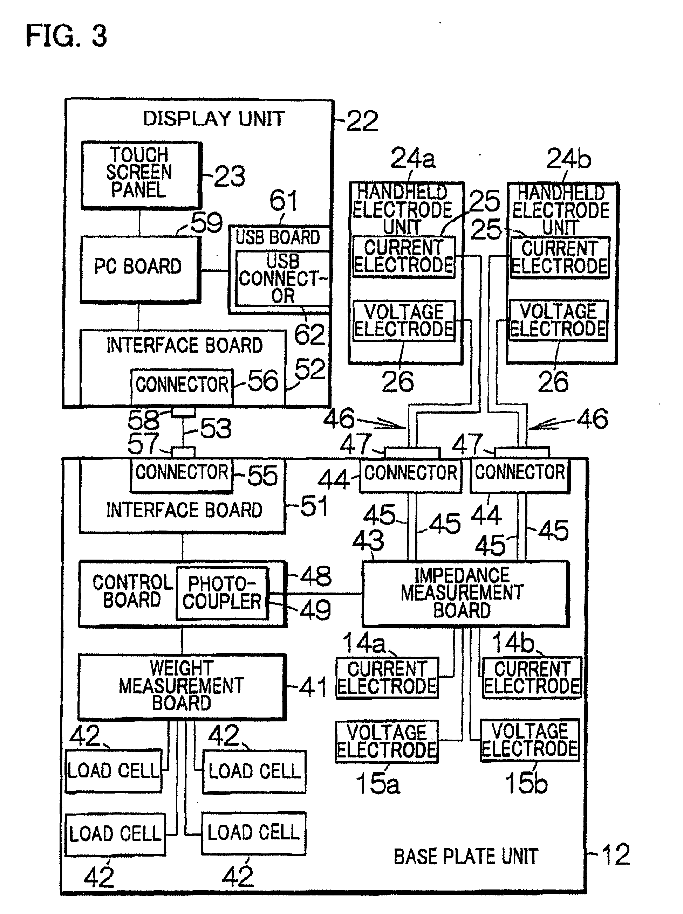

As shown in Fig. 3, a weight measurement circuit, i.e., weight measurement board 41, is built in base plate unit 12. Load cells (weight-to-signal conversion device) 42 in load cell unit 31 are connected to weight measurement board 41. Electric signals of load cells 42 are supplied to weight measurement board 41. Based on the supplied electric signals, weight measurement board 41 identifies a weight value imposed on platform 13. A weight value is an example of measurement values obtained from a user, or a living body, or a human subject. Weight measurement board 41 generates a measurement value data indicating a weight value, i.e., a weight signal.

An impedance measurement circuit, i.e., an impedance measurement board 43 is built in base plate unit 12. Connected to impedance measurement board 43 are current electrodes 14a and 14b and voltage electrodes 15a, and 15b provided on platform 13 and current electrodes 25 and 25 and voltage electrodes 26 and 26 in handheld electrode units 24a and 24b. Impedance measurement board 43 supplies a living body, i.e., a user, with an alternating current of the predetermined frequency by way of a pair of current electrodes selected from among current electrodes 14a, 14b, 25, and 25. In other words, a current pathway is formed through a pair of either two of current electrodes 14a, 14b, 26, and 26 and the living body. In order to measure impedance of various parts of a living body, a selected pair of current electrodes may be switched from one to another while repeating current supply.

Impedance measurement board 43, in response to the supply of the alternating current, measures voltage of a living body by using a pair of selected electrodes from among voltage electrodes 15a, 15b, 26, and 26. In order to measure impedance of various parts of a living body, a selected pair of voltage electrodes may be switched from one to another while repeating voltage measurements. An impedance value is calculated from a current value and a voltage value. The value of impedance is another example of measurement values obtained from a user, i.e., a living body. Impedance measurement board 43 generates measurement value data showing an impedance value, i.e., an impedance signal.

Connector 44 is mounted on base plate unit 12, the connector 44 for connecting impedance measurement board 43, current electrode 25, and voltage electrode 26. Connector 44 is connected to impedance measurement board 43. A connection between connector 44 and impedance measurement board 43 is a signal line 45 for each current electrode 25 and each voltage electrode 26. On the other hand, for example, a single cable 46 is connected to respective handheld electrode units 24a and 24b. Inside each cable 46 are signal lines for respective current electrode 25 and voltage electrode 26. One end of each cable 46 is connected to a connector 47. When connectors 44 and 47 are connected to each other, an independent signal line is established froom each current electrode 25 and each voltage electrode 26 to impedance measurement board 43. Thus, each handheld electrode unit 24a and 24b can be connected to base plate unit 12 by being attachable and detachable by the action of connectors 44 and 47.

A control board (control circuit) 48 is built within base plate unit 12. Connected to control board 48 are a weight measurement board 41 and an impedance measurement board 43. Control board 48 obtains, as pieces of measurement value data, a weight signal and an impedance signal from weight measurement board 41 and impedance measurement board 43. Provided on control board 48 is a calculation processing circuit. The calculation processing circuit calculates a living body index for a living body in accordance with a predetermined calculation method.

A living body index to be calculated includes an index related to the body composition of a living body. An index related to the body composition includes subcutaneous fat thickness, abdominal muscle thickness, subcutaneous fat area, visceral fat area, abdominal total fat area, trunk portion fat ratio, and whole body fat ratio. The above-described weight value and impedance value are used for calculation of such an index. A method of calculation is set based on, for example, a software computer program. A software computer program may be stored, for example, in a non-volatile memory (not shown). Such a non-volatile memory may be connected to the calculation processing circuit.

For connecting impedance measurement board 43 to control board 48, a photocoupler 49 is mounted on control board 48. A signal line (not shown) from the calculation processing circuit is connected to photocoupler 49. Similarly, another signal line (not shown) is connected from impedance measurement board 43 to photocoupler 49. Photocoupler 49 transmits a signal firm impedance measurement board 43 to control board 43 by means of light. Impedance measurement board 43 and control board 43 are electrically insulated by the action of photocoupler 49.

Display unit 22 is connected to control board 48. For this connection, an interface board 51 (second interface board) is built into base plate unit 12. An interface board 52 (first interface board) is also built into display unit 22. Interface boards 51 and 52 are connected via cable 53. A connector 55 (second connector) and a connector 56 (first connector) are mounted to interface bards 51and 52, respectively. The two ends of cable 53 are a connector 57 (fourth connector) and a connector 58 (third connector). Connector 57 is connected to connector 55 by being attachable and detachable therefrom, and connector 58 is connected to connector 56 by being attachable and detachable therefrom. Thus, a signal line is established between interface boards 51 and 52. Each of interface boards 51 and 52 may use, for example, an RS232 interface. Alternatively, a wireless interface for wireless communication may be used instead of a wired interface such as above.

A PC board (personal computer board) (processor) 59 is built in display unit 22. Interface board 52 and touch screen panel 23 are connected to PC board 59. PC board 59 controls an operation of touch screen panel 23. A signal is exchanged between PC board 59 and control board 48 by the action of interface board 52.

A USB board 61 is connected to PC board 59. A USB connector 62 is mounted on USB board 61. Various types of peripheral devices such as a USB memory can be connected to USB connector 62. Information indicating a living body index can be output via USB connector 62 to a USB memory or other types of peripheral devices. In this embodiment, USB board 61 is provided on display unit 22, but may be provided on base plate unit 12.

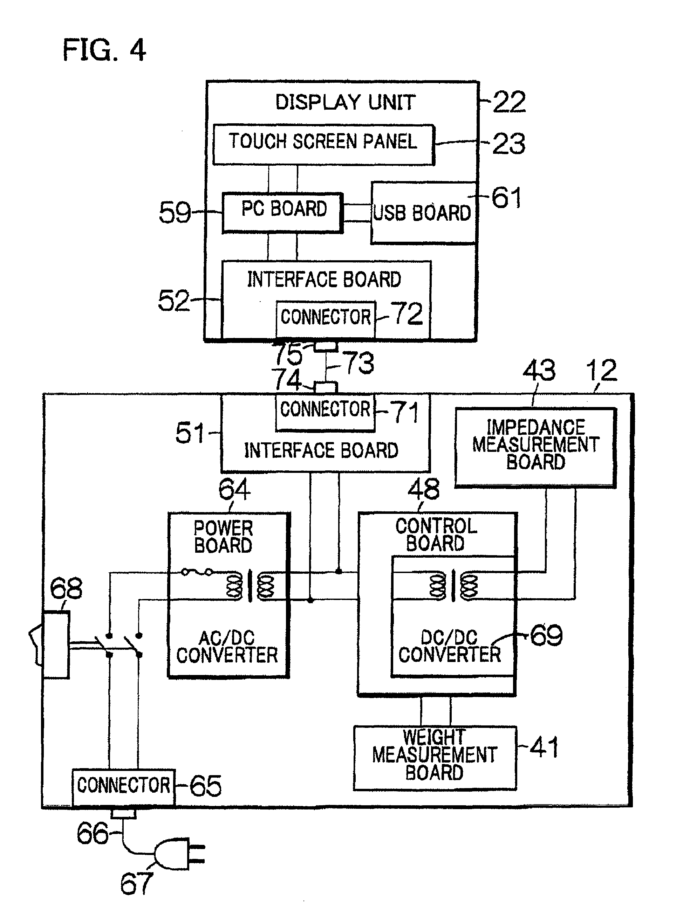

As shown in Fig. 4, a power board 64 is built into base plate unit 12. An AC/DC converter is mounted on power board 64. The primary circuit of the AC/CD converter is connected to power source connector 65. Power source connector 65 is exposed on the lateral side of base plate unit 12. A power source code 66 is connected to power source connector 65. The other end of power source code 66 is coupled with a plug 67. Thus, an alternating current voltage is supplied to the primary circuit from a power source. The secondary circuit of the ACIDS converter is connected to control board 48. The AC/DC converter converts the alternating current voltage into a direct current voltage. The direct current voltage is supplied to control board 48. By the action of the AC/DC converter, for example, a commercial voltage of 230V is converted to a direct current voltage of 12V.

A power source switch 68 is connected between power source connector 65 and power board 64. Power source switch 68 controls the continuity and the break of electricity between power source connector 65 and power board 64. Power source switch 68 is exposed on the lateral side of base plate unit 12.

Electricity is supplied from control board 48 to weight measurement board 41 and impedance measurement board 43. For the supply to impedance measurement board 43, a DC/DC converter 69 is mounted on control board 48. DC/DC converter 69 converts a voltage of a predetermined voltage value to a voltage of a smaller voltage value. A predetermined voltage supplied from control board 48 is supplied to weight measurement board 41. A voltage having a voltage value smaller than the predetermined voltage value supplied from control board 48 is supplied to impedance measurement board 43. DC/DC converter 69 converts, for example, a direct current voltage of 12V into a direct current voltage of 5V_{.}

Display unit 22 is connected to power board 64. Power source connectors 71 and 72 are mounted on the above described interface boards 51 and 52, respectively. Power source connectors 71 and 72 are mutually connected by a power source cable 73. Coupled to the two ends of power source cable 73 are power source connectors 74 and 75. Power source connectors 74 and 75 are connected to power source connectors 71 and 72, respectively, by each being freely attachable and detachable. Thus, a power source line is established between interface boards 51 and 52. By the action of interface boards 51 and 52, electricity is supplied from power board 64 to PC board 59. The electricity is supplied from PC board 59 to touch screen panel 23 and USB board 61

We assume a situation in which a user measures a living body index. The user stands upright on platform 13, and instructs a measurement from touch screen panel 23. For example, the user touches touch screen panel 23 using a finger. A specifies measurement item is selected based on the touch. PC board 59 instructs control board 48 for a measurement depending on the measurement item. The user grasps handheld electrode units 24a and 24b if the measurement of impedance by means of handheld electrode units 24a and 24b is needed for the measurement of the selected measurement item. For example, impedance measurement board 43 supplies an alternating current to a user by means of a particular pair of electrodes from among current electrodes 14a, 14b, 25, and 25. Subsequently, impedance measurement board 43 measures the voltage of a user by way of a particular pair of electrodes from among voltage electrodes 15a, 15b, 26, and 26. Impedance measurement board 43 calculates an impedance value of the user. Control board 48 calculates a living body index for the measurement item based on the impedance value and the weight value. Information showing the living body index is transmitted to PC board 59. Information showing the living body index is then displayed on touch screen panel 23.

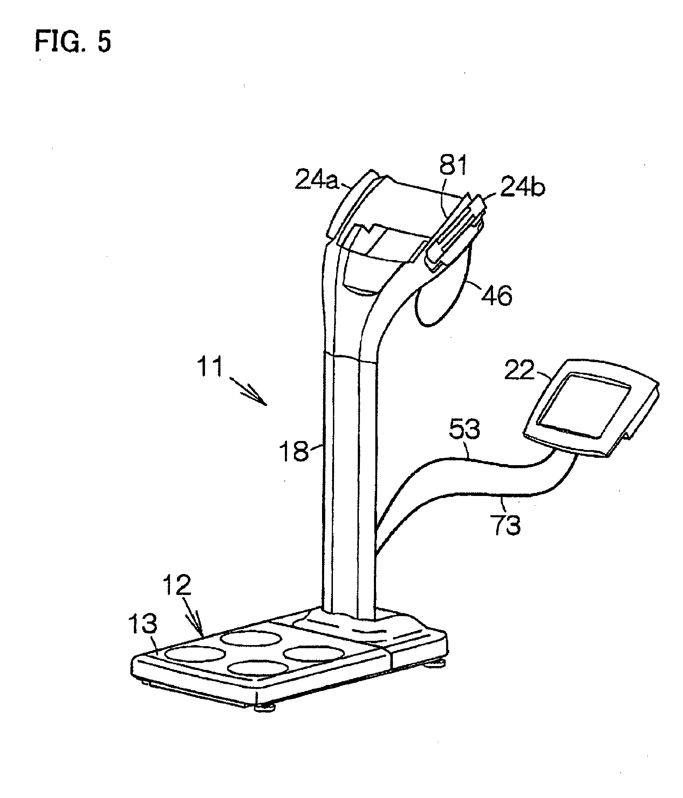

As shown in Fig. 5, according to this body composition measurement apparatus 11, display unit 22 can be detached from pillar 8. Display unit 22 only has to be received in a recess of pillar 18, and may be recessed by the elastic force. In this way, cable 46, cable 53, and power source cable 73 are extended toward the outside of pillar 18. Therefore, the connection between display unit 22 and base plate unit 12 can be maintained even if display unit 22 is detached. A measure other than the user himself who is on platform 13 is allowed to operate touch screen panel 23. A measurer other than the user himself who is on platform 13 is allowed to view a display on touch screen panel 23. In this case, a measurer other than the user himself who is on platform 13 can distance the operation and the display from the eyes of the user.

Furthermore, display unit 22 and cable 53, and display unit 22 and power source cable 73 are connected by being freely attachable and detachable from each other. Therefore, display unit 22 can be easily replaced at the installed site. Touch screen panel 23 or PC board 59 can be replaced or repaired at a place outside the installed site. The entire body composition measurement apparatus 11 does not have to be transported for such repairs and replacements. Therefore, the work burden is reduced. The workability is enhanced in repairs and replacements. PC board 59 and touch screen panel 23 can be updated relatively easily.

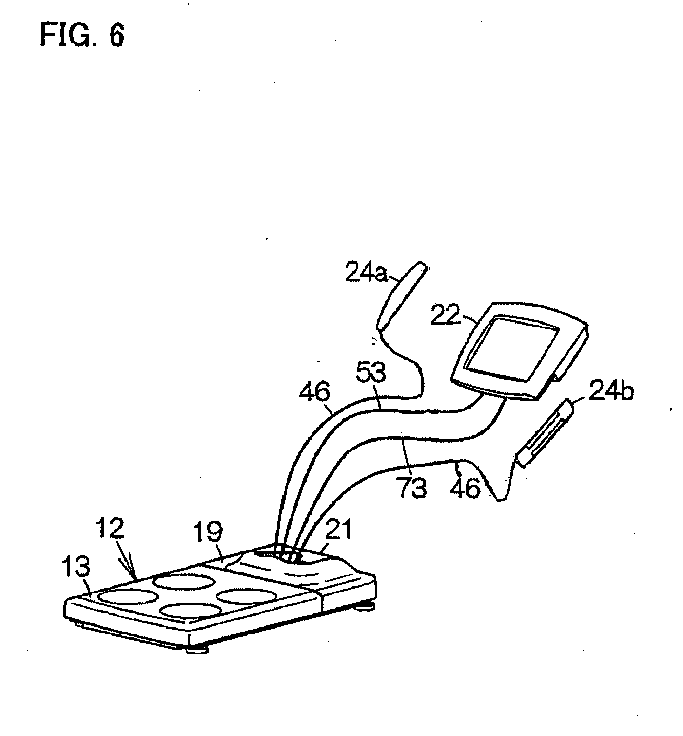

As shown in Fig. 6, according to body composition measurement apparatus 11, pillar 18 can be removed. Prior to the removal, front cover 19 and rear cover 21 are removed from base 16. Pillar 18 can be removed from base 15 when finger screw 28 is loosened and removed. In this case, interface board 51 is left in base plate unit 12. Therefore, the connection between display unit 22 and base plate unit 12 can be maintained. After pillar 18 is removed, front cover 19 and rear cover 21 are put back to base 16, by attaching thereto. A user, even without pillar 18, can view touch screen panel 23 while grasping handheld electrode units 24a and 24b.

When pillar 18 is removed, cable 46, cable 53, and power source cable 73 can be removed from base plate unit 12. Therefore, base plate unit 12 can be more easily carried in comparison with a conventional device. Repairs and replacements of control board 48, weight measurement board 41., impedance measurement board 43, or other components can be performed relatively easily. The workability is enhanced in repairs and replacements.

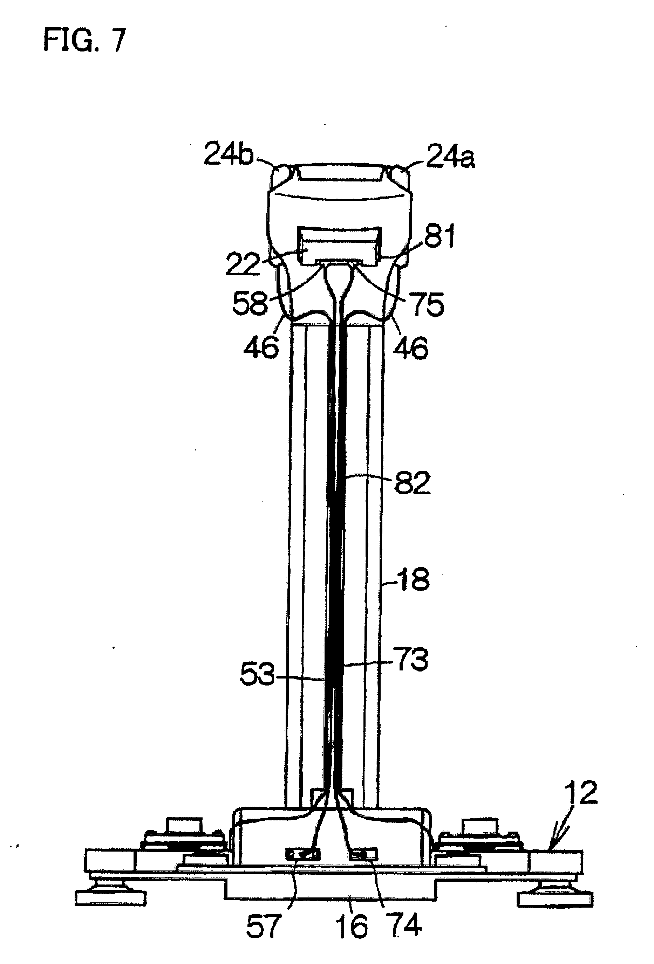

As shown in Fig. 7, a groove 82 extending in the vertical direction may be formed on the back side of pillar 18. Groove 82 at least has to extend, for example, from the root of pillar 18 to a recess 81. Groove 82 accommodates cable 46, cable 53, and power source cable 73. As a result, cable 46, cable 53, and power source cable 73 are allowed to extend along the outside surface of pillar 18 between base plate unit 12 and display unit 22. Alternatively, cable 46, cable 53, and power source cable 73 may run along the outside surface of pillar 18 without having groove 82. In this case, a clip or the like may be used for fixing of cable 46, cable 53, and power source cable 73 to pillar 18.

In interface board 51, connector 55 and power source connector 71 may be integrated as a single component. Similarly, in interface board 52, connector 56 and power source connector 72 may be integrated as a single component. In such cases, cable 46, cable 53, and power source cable 73 may be accommodated by a single sheath. Furthermore, connector 57 and power source connector 74 may be integrated as a single component, and connector 58 and power source connector 75 may be integrated as a single component.

In the above embodiment, handheld electrode units 24a and 24b are provided with body composition measurement apparatus 11, but handheld electrode units 24a and 24b do not have to be provided. In this case, impedance (foot-to-foot impedance) of a living body is measured by using electrodes 14a, 14b, 15a, and 15b, and the measured impedance is used for calculation of a living body index. In this case, because cable 46 is no longer necessary, only cable 53 and power source cable 73 may be accommodated by a single sheath in a case in which connector 55 and power source connector 71 of interface board 51 are integrated as a single component, and in which connector 56 and power source connector 72 of interface board 52 are integrated as a single component.

## Claims

1. A living body index measurement apparatus (11) for measuring a living body index of a human subject and for displaying information on the measured living body index, comprising:
a base plate unit (12) having a platform (13) on which the human subject stands while measurement is taken;
a calculation processing circuit (48) that is built in the base plate unit and for calculating a living body index of the human subject based on a measurement value obtained from the human subject;
a pillar (18) that stands upright from the base plate unit, with a bottom end of the pillar being connected detachably to the base plate unit;
a display unit (22) that is detachably mounted at a top end of the pillar and for displaying information including the living body index, the display unit having a processor (59); and
a cable (53) for connecting the display unit and the base plate unit, **characterised in that**:
the pillar (18) has a back side in which a groove (82) is formed, the groove (82) extending in the vertical direction,
the display unit (22) is received in a recess at a top end of the pillar (18) in a detachable manner, and includes a first interface board (52) having a first connector (56) connected to the processor (59),
a second interface board (51) is built in the base plate unit (12), the second interface board (51) having a second connector (55) connected to the calculation processing circuit (48) in the base plate unit (12),
the cable (53) has an end having a third connector (58) detachably connected to the first connector (56) of the first interface board (52) in the display unit (22), the cable (53) having another end having a fourth connector (57) detachably connected to the second connector (55) of the second interface board (51) in the base plate unit (12), the cable (53) extending along the external surface of the pillar (18) and accommodated in the groove (82) of the pillar (18).

2. A living body index measurement apparatus according to Claim 1, further comprising a finger screw (28) for connecting the pillar and the base plate unit, wherein the pillar is connected to the base plate unit in such a manner that is attachable to or detachable from the base plate unit by means of the finder screw.

3. A living body index measurement apparatus according to Claim 1, further comprising:
a weight-to-signal conversion device (42) that is built within the base plate unit and for generating a signal that changes in accordance with a weight of a living body that is on the platform;
a weight measurement circuit (41) that is built within the base plate unit and for generating a weight signal indicating a value of the weight based on the signal from the weight-to-signal conversion device;
plural current electrodes (14a,14b, 25) for supplying a current with the living body;
plural voltage electrodes (15a,15b,26) for contacting the living body; and
an impedance measurement circuit (43) that is built within the base plate unit and for generating an impedance signal indicating an impedance of the living body based on a voltage given to the voltage electrode and the current,
wherein the calculation processing circuit (48) calculates a living body index for the living body based on the weight signal and the impedance signal.

## Patentansprüche

1. Eine Vorrichtung zum Messen des Index eines lebenden Körpers (11) zum Messen des Index eines lebenden Körpers eines menschlichen Individuums und zum Anzeigen von Informationen über den gemessenen Index eines lebenden Körpers, die Folgendes beinhaltet:
eine Bodenplatteneinheit (12) mit einer Plattform (13), auf der das menschliche Individuum steht, während die Messung vorgenommen wird;
eine Berechnungsverarbeitungsschaltung (48), die in die Bodenplatteneinheit eingebaut ist, und zum Berechnen eines Indexes eines lebenden Körpers des menschlichen Individuums auf der Grundlage eines von dem menschlichen Individuum erhaltenen Messwerts;
eine Säule (18), die aufrecht von der Bodenplatteneinheit steht, wobei das untere Ende der Säule abnehmbar mit der Bodenplatteneinheit verbunden ist; und
eine Anzeigeeinheit (22), die abnehmbar am oberen Ende der Säule montiert ist, und zum Anzeigen von Informationen, einschließlich des gemessenen Index eines lebenden Körpers, wobei die Anzeigeeinheit einen Prozessor (59) aufweist; und
ein Kabel (53) zum Verbinden der Anzeigeeinheit und der Bodenplatteneinheit,
**dadurch gekennzeichnet, dass**:
die Säule (18) eine Rückseite aufweist, in der eine Rille (82) ausgebildet ist, wobei die Rille (82) sich in senkrechter Richtung erstreckt,
die Anzeigeeinheit (22) auf abnehmbare Weise in einer Vertiefung am oberen Ende der Säule (18) aufgenommen wird und eine Schnittstellenplatine (52) mit einem ersten Steckverbinder (56), der mit dem Prozessor (59) verbunden ist, umfasst,
eine zweite Schnittstellenplatine (51) in die Bodenplatteneinheit (12) eingebaut ist, wobei die zweite Schnittstellenplatine (51) einen zweiten Steckverbinder (55) aufweist, der mit der Berechnungsverarbeitungsschaltung (48) in der Bodenplatteneinheit (12) verbunden ist;
das Kabel (53) ein Ende aufweist, das einen dritten Steckverbinder (58) aufweist, der abnehmbar mit dem ersten Steckverbinder (56) der ersten Schnittstellenplatine (52) in der Anzeigeeinheit (22) verbunden ist, wobei das Kabel (53) ein weiteres Ende aufweist, das einen vierten Steckverbinder (57) aufweist, der abnehmbar mit dem zweiten Steckverbinder (55) der zweiten Schnittstellenplatine (51) der Bodenplatteneinheit (12) verbunden ist, wobei das Kabel (53) sich an der Außenfläche der Säule (18) entlang ersteckt und in der Rille (82) der Säule (18) untergebracht ist.

2. Eine Vorrichtung zum Messen des Index eines lebenden Körpers gemäß Anspruch 1, der ferner eine Fingerschraube (28) zum Verbinden der Säule und der Bodenplatteneinheit beinhaltet, wobei die Säule auf eine solche Weise mit der Bodenplatteneinheit verbunden ist, dass sie mithilfe der Fingerschraube an der Bodenplatteneinheit befestigt werden kann oder von dieser abgenommen werden kann.

3. Eine Vorrichtung zum Messen des Index eines lebenden Körpers gemäß Anspruch 1, die ferner Folgendes beinhaltet:
ein Gewicht-zu-Signal-Umwandlungsgerät (42), das innerhalb der Bodenplatteneinheit eingebaut ist, und zum Erzeugen eines Signals, das sich gemäß dem Gewicht eines lebenden Körpers, der sich auf der Plattform befindet, ändert;
eine Gewichtsmessungsschaltung (41), die innerhalb der Bodenplatteneinheit eingebaut ist, und zum Erzeugen eines Gewichtssignals, das einen Wert des Gewichts auf der Grundlage des Signals von dem Gewicht-zu-Signal-Umwandlungsgerät angibt;
mehrere Stromelektroden (14a, 14b, 25) zum Versorgen des lebenden Körpers mit einem Strom;
mehrere Spannungselektroden (15a, 15b, 26) zum Kontaktieren des lebenden Körpers; und
eine Impedanzmessungsschaltung (43), die innerhalb der Bodenplatteneinheit eingebaut ist, und zum Erzeugen eines Impedanzsignals, das eine Impedanz des lebenden Körpers auf der Grundlage einer zur Spannungselektrode ausgegebenen Spannung und des Stroms angibt,
wobei die Berechnungsverarbeitungsschaltung (48) einen Index eines lebenden Körpers für den lebenden Körper auf der Grundlage des Gewichtssignals und des Impedanzsignals berechnet.

## Revendications

1. Un appareil de mesure d'un indice de corps vivant (11) destiné à mesurer un indice de corps vivant chez un sujet humain et à afficher l'information sur l'indice de corps vivant mesuré, comprenant :
Un socle (12) doté d'une plateforme (13) sur laquelle le sujet humain se tient debout pendant que la mesure est prise :
Un circuit de traitement (48) intégré au socle qui calcule l'indice de corps vivant du sujet humain sur la base d'une valeur de mesure obtenue à partir du sujet humain ;
Une colonne (18) qui se tient droite à partir du socle, dotée d'une extrémité inférieure reliée au socle et détachable de celui-ci ;
Un affichage (22) détachable monté sur l'extrémité supérieure de la colonne destiné à afficher l'information dont l'indice de corps vivant, cet affichage étant doté d'un processeur (59) ; et
Un câble (53) reliant l'affichage et le socle, **caractérisé en ce que** :
La colonne (18) est dotée d'une rainure (82) à l'arrière, cette rainure (82) s'étendant de manière verticale,
L'affichage (22) est détachable et inséré dans un renfoncement situé à l'extrémité supérieure de la colonne (18), et comprend une première carte d'interface (52) dotée d'un premier connecteur (56) relié au processeur (59),
Une seconde carte d'interface (51) est intégrée au socle (12), cette seconde carte d'interface (51) étant dotée d'un second connecteur (55) relié au circuit de traitement (48) effectuant le calcul, lequel se trouve dans le socle (12),
Le câble (53) est doté à une extrémité d'un troisième connecteur (58) détachable relié au premier connecteur (56) de la première carte d'interface (52) de l'affichage (22), le câble (53) étant doté à une autre extrémité d'un quatrième connecteur (57) détachable et relié au second connecteur (55) de la seconde carte d'interface (51) dans le socle (12), le câble (53) s'étendant le long de la surface externe de la colonne (18) dans la rainure (82) de la colonne (18).

2. Un appareil de mesure d'un indice de corps vivant selon la revendication 1, comprenant, en outre, une vis de serrage (28) pour relier la colonne au socle, dans lequel la colonne est reliée au socle de manière à ce qu'elle puisse y être fixée ou en être détachée à l'aide de la vis de serrage.

3. Un appareil de mesure d'un indice de corps vivant selon la revendication 1, comprenant, en outre :
un appareil de conversion du poids en signal (42) intégré au socle permettant de générer un signal qui change en fonction du poids d'un corps vivant situé sur la plateforme ;
un circuit de mesure du poids (41) intégré au socle, permettant de générer un signal de pesée indiquant une valeur de poids fondée sur le signal de l'appareil de conversion du poids en signal ;
plusieurs électrodes de courant (14a, 14b, 25) pour approvisionner le corps vivant en courant ;
plusieurs électrodes de tension (15a, 15b, 26) permettant le contact avec le corps vivant ; et
un circuit de mesure de l'impédance (43) intégré au socle, permettant de générer un signal d'impédance indiquant une impédance du corps vivant fondée sur une tension donnée à l'électrode de tension et au courant,
dans lequel un circuit de traitement (48) calcule un indice de corps vivant pour le corps vivant ; cet indice étant fondé sur le signal de pesée et sur le signal d'impédance.
